# EUROPEAN PATENT SPECIFICATION

(11) **EP 4 281 432 B1**
(45) Date of publication and mention of the grant of the patent: **11.03.2026**
(21) Application number: 22742375.3
(22) Date of filing: 19.01.2022
(51) Int. Cl.: C07C 51/43, C07C 51/02, C12M 1/36, C12P 41/00, C12N 9/04, B01D 9/00

(54) **CRYSTALLIZATION OF HIGH-PURITY MAGNESIUM L-LACTATE**
KRISTALLISIERUNG VON HOCHREINEM MAGNESIUM-L-LACTAT
CRISTALLISATION DE L-LACTATE DE MAGNÉSIUM DE HAUTE PURETÉ

(30) Priority: 21.01.2021 US 202163139894 P; 17.11.2021 US 202163280150 P
(43) Date of publication of application: 29.11.2023
(73) Proprietor: TripleW Ltd., 4250407 Netanya (IL)
(72) Inventor: GREENER, Tsvika, 7406505 Ness Ziona (IL); PAPO, Nitsan, 4527314 Hod Hasharon (IL); HARNOY, Assaf, 4651630 Herzliya (IL)
(74) Representative: Pearl Cohen EU
(86) International application number: PCT/IL2022/050081
(87) International publication number: WO 2022/157768

(56) References cited:
- EP-A1- 2 604 696
- WO-A1-2020/110108
- WO-A1-2020/110108
- CN-A- 104 789 607
- CN-B- 105 018 538

## Description

### FIELD OF THE INVENTION

The present invention relates to a process for the crystallization of high purity magnesium L-lactate from decomposed organic waste and/or enriching L-lactate enantiomer from an enantiomeric mixture of D- and L-lactate derived from decomposed organic waste.

### BACKGROUND OF THE INVENTION

Lactic acid is the most widely occurring hydroxycarboxylic acid with applications in food, chemical, pharmaceutical, and cosmetic industries. This naturally occurring organic acid can be produced by chemical synthesis or microbial fermentation. When produced by microbial fermentation, care should be taken to avoid endogenous decrease in pH due to the formation of lactic acid in order to maintain the productivity of the microorganisms. A pH in the range of 5-7 is preferable and can be obtained by the addition of bases such as ammonium -, sodium -, potassium -, magnesium - or calcium hydroxides that neutralize the lactic acid thereby producing a lactate salt. In order to convert the lactate salt to lactic acid, a re-acidification step using e.g., sulfuric acid can be performed.

Approximately 60-80% of the production costs of lactic acid can result from downstream processes, including the purification, concentration, and separation of the lactic acid/lactate salt from the fermentation broth. Additionally, reducing or eliminating the production of by-products (such as salts other than lactate) is desirable.

Various methods have been previously proposed for recovering and purifying lactic acid and/or lactate salts from fermentation broths. For example, a process for magnesium lactate purification based on crystallization is described in Wang Yong, et al., "Efficient magnesium lactate production with in situ product removal by crystallization", Bioresource technology 198 (2015): 658-663. The crystallization was conducted at 42°C without the addition of crystal seeds. The fermentation medium used in the fermentor contained yeast extract, glucose, NaCl, sodium acetate, triammonium citrate, KH₂PO₄, MgSO₄·7H₂O, and MnSO₄·7H₂O. The product concentration, productivity and yield of fermentation coupled with *in situ* product removal (ISPR) reached 143 g L⁻¹, 2.41 g L⁻¹h⁻¹ and 94.3%, respectively.

U.S. Patent No. 9,689,007 and EP 2604696 describe a method for producing lactate or lactic acid from "low sugar" plant extracts via fermentation, comprising providing a fermentation medium that includes at least 25wt.% of a plant extract containing fermentable carbohydrates, and fermenting the fermentation medium by means of a lactic acid producing microorganism in the presence of a caustic magnesium salt to provide a fermentation broth containing at the most 9.5wt.% magnesium lactate at the end of fermentation, the magnesium lactate being in soluble form during and at the end of fermentation. To achieve a magnesium lactate concentration in the fermentation broth at the end of fermentation which is at most 9.5wt.%, the fermentation medium comprising the plant extract preferably contains fermentable carbohydrates in a concentration of at most 9.5wt.%.

U.S. Publication No. 2014/0012041 describes a method of producing a lactic acid salt comprising: subjecting an aqueous lactic acid salt solution comprising a formic acid salt in an amount of not less than 7.0% by weight with respect to said lactic acid salt to crystallization, and recovering said lactic acid salt. The lactic acid salt concentration in said aqueous lactic acid salt solution is 10.0 to 30.0% by weight.

U.S. Publication No. 2017/0218408 describes a method for preparing a fermentation product including lactic acid, the method including: a) treating a lignocellulosic material, being in a particulate state and having an average particle size of from 0.1 to 250 mm, with caustic magnesium salt in the presence of water to provide treated aqueous lignocellulosic material; b) saccharifying the treated aqueous lignocellulosic material in the presence of a hydrolytic enzyme to provide a saccharified aqueous lignocellulosic material comprising fermentable carbohydrate and a solid lignocellulosic fraction; c) simultaneously with step b), fermenting the saccharified aqueous lignocellulosic material in the presence of both a lactic acid forming microorganism and caustic magnesium salt to provide an aqueous fermentation broth comprising magnesium lactate and a solid lignocellulosic fraction; d) recovering magnesium lactate from the broth, wherein the saccharification and the fermentation are carried out simultaneously. The feedstock for the process of U.S. Publication No. 2017/0218408 is a lignocellulosic material, which includes materials containing cellulose, hemicellulose and lignin, such as may be derived from plant biomass. Preferred lignocellulosic materials are selected from the group consisting of: wheat straw, sugarcane bagasse, corn stover, and mixtures thereof.

WO Publication No. 2017/178426 describes a fermentation process for producing magnesium lactate from a carbon source comprising the steps of:
- providing a fermentation medium comprising a fermentable carbon source in a fermentation reactor,
- fermenting the fermentation medium by means of a lactic acid producing microorganism in the presence of an alkaline magnesium salt to provide a fermentation broth comprising magnesium lactate, and
- recovering solid magnesium lactate from the magnesium lactate containing fermentation broth, wherein during at least 40% of the operating time of the fermentation process, the concentration of solid magnesium lactate in the fermentation broth is maintained in the range of 5-40 vol.%, calculated as solid magnesium lactate crystals on the total of the fermentation broth. Examples of fermentable carbon sources are C₅ sugars, C₆ sugars, oligomers thereof (e.g. dimeric C₁₂ sugars) and/or polymers thereof.

WO Publication No. 2017/207501 describes a method for separating biomass from solid fermentation product wherein a slurry comprising biomass and solid fermentation product is provided to the top of a biomass separator unit and an aqueous medium is provided to the bottom of a biomass separator unit, while a product stream comprising solid fermentation product is withdrawn from the bottom of the biomass separator unit and a waste stream comprising biomass is withdrawn from the top of the biomass separator unit. The solid fermentation product is a fermentation product which is present in the aqueous medium in a concentration above its saturation concentration, and may be a crystalline product or an amorphous product.

The above-described purification and crystallization methods are designed for fermentation broths derived from substantially homogenous biomass-based feed streams having low soluble and insoluble impurities content. There is a need, however, to utilize more available and less expensive non-homogeneous feedstocks for fermentation, such as mixed food waste from municipal, industrial and commercial origins. These non-homogeneous feedstocks contain impurities such as salts, lipids, proteins, color components, and inert materials. Fermentation broths derived from said non-homogeneous feedstocks cannot be effectively processed by the currently available methods in order to obtain a high-purity magnesium L-lactate product in a cost-effective manner.

WO Publication No. 2020/110108 describes a process for the separation and purification of magnesium lactate from a fermentation broth, comprising the steps of:
(a) providing a clarified fermentation broth from which insoluble impurities have been removed comprising magnesium lactate in a soluble form being the result of a fermentation process, the fermentation broth being at a temperature of about 45°C to about 75°C;
(b) concentrating the clarified broth from step (a) to a concentration of about 150 g/L to about 220 g/L of lactate;
(c) performing at least one cooling crystallization of the concentrated clarified broth from step (b) to obtain magnesium lactate crystals; and
(d) collecting the magnesium lactate crystals obtained.

CN 105018538 describes a magnesium lactate production method based on crystallization process for fermentation, separation and coupling. *Lactobacillus rhamnosus* is subjected to repeated batch fermentation culture for 180-220 h manner at 37-42°C, and *in-situ* separation is performed to obtain the high-purity magnesium lactate crystals.

CN 104789607 describes a method for preparing a lactic acid and/or lactate through fermentation-separation coupling. According to the method, starch is used as fermenting materials; magnesium salt is used as a neutralizing agent for fermentation production of the lactic acid; the fermentation-separation coupling technique is utilized for separation and purification so as to obtain the lactic acid or lactate.

There remains an unmet need for simple, cost-effective methods with high recovery yields for the crystallization of high purity magnesium L-lactate salt from decomposed organic waste.

### SUMMARY OF THE INVENTION

The present invention is set out in the appended set of claims.

In particular, there is provided a process for preparing high-purity magnesium L-lactate from fermentation broths or other organic waste decomposition products containing lactic acid and/or lactate salts. The process comprises evaporative crystallization which can utilize fermentation broths or other organic waste decomposition products from diverse origins thereby rendering the process both economically and environmentally beneficial. The process is suitable for fed batch production as well as continuous production in lactic acid recycling facilities.

Unexpectedly, high-purity magnesium L-lactate crystals were found to be obtained from dispersions of decomposed organic waste such as lactic acid fermentation broths without the necessity of removing D-lactic acid during and/or after fermentation. The crystals are formed by evaporative crystallization under specific conditions which yields high-purity magnesium L-lactate already after a single pass despite the presence of significant amounts of impurities in the decomposed waste. Surprisingly, an improved enantiomeric separation even when using decomposed organic waste containing endogenous D-lactic acid of up to 10wt.% was obtained. The process is therefore simple and economic providing high purity magnesium L-lactate crystals even when a non-homogeneous feedstock comprising high concentrations of soluble and insoluble impurities from municipal, industrial, and commercial origins is used.

According to a first aspect, the present invention provides a process for the formation of high-purity magnesium L-lactate crystals from decomposed organic waste and/or enriching L-lactate enantiomer from an enantiomeric mixture of D- and L-lactate derived from decomposed organic waste, the process comprising the steps of:
a. providing a clarified dispersion of decomposed organic waste comprising a lactate salt in a concentration of about 50 to about 110 g/L;
b. optionally concentrating the clarified dispersion of step (a) to a lactate salt concentration of about 100 to about 150 g/L;
c. mixing the clarified dispersion of step (a) or the concentrated clarified dispersion of step (b) to obtain a suspension comprising seed magnesium L-lactate crystals;
d. removing from about 70% to about 90% of water from the suspension of step (c) to obtain magnesium L-lactate crystals at high purity and/or enriched enantiomeric purity; and
e. collecting the magnesium L-lactate crystals obtained in step (d),
wherein steps (b) through (d) are performed at elevated temperature in the range of about 50°C to about 90°C and the application of a vacuum to a pressure of about 80 to about 300 mbar, including each value within the specified ranges.

The decomposed organic waste may be obtained from a lactic acid fermentation process or from a lactic acid-containing waste. According to an example, the decomposed organic waste is obtained from hydrolysis of polylactic acid polymer.

According to some embodiments, the clarified dispersion of decomposed organic waste comprises decomposed organic waste from which impurities have been removed using at least one of filtration, centrifugation, flotation, sedimentation, coagulation, flocculation, and decantation.

According to further embodiments, the organic waste is selected from food waste, municipal food waste, residential food waste, agricultural waste, industrial food waste from food processing facilities, commercial food waste, and a mixture or combination thereof.

According to other embodiments, the decomposed organic waste comprises a fermentation broth, preferably wherein the fermentation broth is obtained from a fermentation process of a carbohydrate source or wherein the fermentation broth is obtained from a fermentation process of an organic waste feedstock.

According to some embodiments, step (b) is performed to a lactate salt concentration of about 100 to about 130 g/L.

According to various embodiments, the mixing in step (c) is performed at a speed of about 50 to about 300 rounds per minute (RPM).

According to certain embodiments, the mixing in step (c) is performed for at least 1 hour.

According to further embodiments, steps (b) through (d) are performed at elevated temperature in the range of about 50°C to about 80°C. According to other embodiments, steps (b) through (d) are performed with the application of a vacuum to a pressure of about 150 to about 250 mbar. According to further embodiments, steps (b) through (d) are performed with the application of a vacuum to a pressure of about 250 to about 350 mbar.

According to particular embodiments, removing from about 70% to about 90% of water from the suspension in step (d) is performed at an evaporation rate of loss of about 2 to about 5 wt% of the total weight of the suspension per hour.

According to various embodiments, step (e) comprises filtration and/or centrifugation. According to specific embodiments, step (e) is performed at room temperatures.

According to certain embodiments, the process further comprises step (f) of washing the obtained magnesium L-lactate crystals. According to particular embodiments, washing of the obtained magnesium L-lactate crystals is performed in a solvent selected from water, ethanol, propanol, isobutanol, cyclohexane, acetone, ethyl acetate, and a mixture or combination thereof.

According to other embodiments, the process further comprises step (g) of drying the magnesium L-lactate crystals to a Loss on Drying (LOD) % of about 10% to about 20%. According to particular embodiments, step (g) is performed at elevated temperatures of about 50°C to about 120°C.

According to additional embodiments, the obtained magnesium L-lactate crystals are solubilized and re-crystalized by reiterating steps (c) to (e). For example, steps (c) to (e) may be performed in cycles of between two and six cycles.

According to further embodiments, the recovery of magnesium L-lactate crystals is at least 90%.

According to other embodiments, the obtained magnesium L-lactate crystals are characterized by a median size which is smaller than 75 µm. According to certain examples, the obtained magnesium L-lactate crystals are characterized by a size distribution comprising a median size in the range of about 20 to about 100 µm. According to yet other embodiments, the obtained magnesium L-lactate crystals are characterized by a median size which is larger than 75 µm. According to additional embodiments, the obtained magnesium L-lactate crystals are characterized by a size distribution comprising a median size in the range of about 100 to about 300 µm.

According to some embodiments, the obtained magnesium L-lactate crystals comprise less than 3% magnesium D-lactate. According to certain embodiments, the obtained magnesium L-lactate crystals comprise less than 2% magnesium D-lactate. According to other embodiments, the obtained magnesium L-lactate crystals comprise less than 1.5% magnesium D-lactate. According to yet other embodiments, the obtained magnesium L-lactate crystals comprise less than 1% magnesium D-lactate.

Further embodiments will become apparent from the detailed description given hereinafter. However, it should be understood that the detailed description and specific examples, while indicating preferred embodiments of the invention, are given by way of illustration only.

### BRIEF DESCRIPTION OF THE FIGURES

**Figure 1** shows the size distribution of magnesium L-lactate crystals obtained according to some embodiments of the present invention.
**Figures 2A-2F** show light microscopy images of magnesium L-lactate crystals obtained according to some embodiments of the present invention that remained on top of sieves having cutoff sizes of 710 µm (2A), 500 µm (2B), 300 µm (2C), 100 µm (2D), and 75 µm (2E). Figure 2F shows a light microscopy image of magnesium L-lactate crystals that passed the 75 µm sieve.
**Figure 3** shows the size distribution of magnesium L-lactate crystals obtained using cooling crystallization.
**Figures 4A-4F** show light microscopy images of magnesium L-lactate crystals obtained using cooling crystallization that remained on top of sieves having cutoff sizes of 710 µm (4A), 500 µm (4B), 300 µm (4C), 100 µm (4D), and 75 µm (4E). Figure 4F shows a light microscopy image of magnesium L-lactate crystals that passed the 75 µm sieve.
**Figure 5** shows the size distribution of magnesium L-lactate crystals obtained according to some embodiments of the present invention following a recrystallization.
**Figure 6** shows the size distribution of magnesium L-lactate crystals obtained using different evaporation rates.
**Figure 7** shows the size distribution of magnesium L-lactate crystals obtained according to some embodiments of the present invention from a fed batch constant volume crystallization.
**Figure 8** shows a light microscopy image of magnesium L-lactate crystals obtained according to some embodiments of the present invention from a fed batch constant volume crystallization.
**Figure 9** shows the size distribution of magnesium L-lactate crystals obtained according to some embodiments of the present invention from a fed batch constant concentration crystallization.
**Figure 10** shows a light microscopy image of magnesium L-lactate crystals obtained according to some embodiments of the present invention from a fed batch constant concentration crystallization.
**Figure 11** shows the size distribution of magnesium L-lactate crystals obtained according to some embodiments of the present invention following a recrystallization of magnesium lactate obtained from decomposed PLA 4032D with sodium hydroxide and counterion replacement.
**Figure 12** shows a light microscopy image of magnesium L-lactate crystals obtained according to some embodiments of the present invention following a recrystallization of magnesium lactate obtained from decomposed PLA 4032D with sodium hydroxide and counterion replacement.
**Figure 13** shows the size distribution of magnesium L-lactate crystals obtained according to some embodiments of the present invention following a recrystallization of magnesium lactate obtained from decomposed PLA with sodium hydroxide and counterion replacement.
**Figure 14** shows a light microscopy image of magnesium L-lactate crystals obtained according to some embodiments of the present invention following a recrystallization of magnesium lactate obtained from decomposed PLA with sodium hydroxide and counterion replacement.
**Figure 15** shows the size distribution of magnesium L-lactate crystals obtained from crystallization of magnesium L-lactate at 30°C.
**Figure 16** shows a light microscopy image of magnesium L-lactate crystals obtained from crystallization of magnesium L-lactate at 30°C.

### DETAILED DESCRIPTION OF THE INVENTION

Provided herein is a process for the formation of magnesium L-lactate crystals from a fermentation broth or other organic waste decomposition products obtainable from feedstocks comprising high concentrations of soluble and insoluble impurities from municipal, industrial, and commercial origins. The process provides L-lactate crystals that exert high overall purity, high enantiomeric purity, can be easily manipulated due to desirable filterability and size distribution and are particularly advantageous for use in subsequent polylactic acid formation.

According to some examples, a clarified dispersion of decomposed organic waste or fermentation broth is obtained, the clarified dispersion or broth comprises lactate ions in a concentration of about 50 to about 110 g/L, including each value within the specified range. Exemplary lactate concentrations include, but are not limited to, about 50, about 55, about 60, about 65, about 70, about 75, about 80, about 85, about 90, about 95, about 100, about 105, or about 110 g/L.

According to certain examples, the dispersion is a decomposition product of any lactic acid-containing waste such as, but not limited to, polylactic acid polymer which was subjected to hydrolysis. According to other examples, a fermentation broth derived from organic waste feedstocks is used. Organic waste feedstocks within the scope of the disclosure can be obtained from any waste source including, but not limited to, food waste, municipal food waste, residential food waste, agricultural waste, industrial food waste from food processing facilities, commercial food waste (from hospitals, restaurants, shopping centers, airports etc.), and a mixture or combination thereof. The organic waste can additionally originate from residues ranging from animal and human excreta, vegetable and fruit residues, plants, cooked food, protein residues, slaughter waste, and combinations thereof. Industrial organic food waste may include factory waste such as by products, factory rejects, market returns or trimmings of inedible food portions (such as skin, fat, crusts and peels). Commercial organic food waste may include waste from shopping malls, restaurants, supermarkets, etc.

According to various examples, the dispersion of decomposed organic waste is a fermentation broth obtained from a fermentation process of a carbohydrate source. When using non-homogenous feedstocks, the dispersion of decomposed organic waste or fermentation broth typically comprises insoluble organic-based impurities such as, but not limited to, microorganisms (e.g. lactic acid producing microorganisms including e.g. yeasts, bacteria and fungi), fats and oils, lipids, aggregated proteins, bone fragments, hair, precipitated salts, cell debris, fibers (e.g. fruit and/or vegetables peels), and residual unprocessed waste (e.g. food shells, seeds, food insoluble particles and debris, etc.). Non-limiting examples of insoluble inorganic-based impurities include plastics, glass, residues from food packaging, sand, and combinations thereof.

Non-limiting examples of soluble impurities include water, solvents, polysaccharides, starch, cellulose, hemicellulose, lignin, seed fragment, salts, color components (e.g. tannins, flavonoids and carotenoids), and combinations thereof. Typically, the soluble and insoluble impurities content of the dispersion or broth is identical to the soluble and insoluble impurities content of the organic waste feedstocks. In some examples, the soluble and insoluble impurities content of the dispersion or broth is lower by at least about 1 wt% compared to the soluble and insoluble impurities content of the organic waste feedstocks. In further examples, the soluble and insoluble impurities content of the dispersion or broth is lower by at least about 5 wt%, about 10 wt%, about 15 wt%, about 20 wt%, about 30 wt%, about 40 wt%, or about 50 wt% compared to the soluble and insoluble impurities content of the organic waste feedstocks.

According to the principles disclosed herein, in order to obtain clarified dispersions of decomposed waste or fermentation broths, the insoluble impurities are removed. The impurities may be removed before and/or after decomposition or fermentation of the waste to produce a clarified liquid. According to certain examples, separation of insoluble impurities is performed prior to decomposition or fermentation. For example, a complex organic waste may be thermally treated, subsequently enzymatically treated, and subsequently a centrifuge, e.g., a decanter centrifuge, removes the bulk of insoluble impurities. The supernatant (which still has some impurities) is pumped into a container or fermenter for lactic acid production.

According to various examples, separating a major portion of the insoluble impurities is performed after decomposition or fermentation. Clarification can be achieved via at least one of filtration, centrifugation, flotation, sedimentation, coagulation, flocculation, and decantation. Typically, the clarified dispersion or broth comprise a dispersion of decomposed organic waste or fermentation broth from which insoluble impurities have been removed using filtration (e.g. microfiltration) and/or centrifugation.

Although not necessary for obtaining clarified dispersion or broth according to the principles disclosed herein, additional removal of impurities can be employed. This includes, for example, product isolation or primary recovery in order to remove or separate extract components that are substantially different from the desired lactic acid product, such as salts and proteins using, for example, ultrafiltration, solvent extraction, salt precipitation, and repulsive extraction. Further purification may be performed in order to remove contaminants with similar physical and chemical properties using crystallization, distillation, evaporation, nanofiltration, reverse osmosis (RO) filtration, solvent extraction, electrodialysis, and diverse types of chromatography (such as adsorption or ion exchange).

Purification methods known in the art are typically designed for fermentation broths derived from substantially homogenous biomass-based feed streams having a relatively low content of solid impurities, and some of said methods utilize crystallization during the fermentation step and/or maintaining the lactic acid salt at a particulate state. Additionally, some of the known methods were designed for use of specific amounts of carbohydrates in the feedstock stream at the beginning of the fermentation process, resulting in specific amounts of magnesium lactate product at the end of the fermentation process. For example, U.S. Patent No. 9,689,007 specifies that the fermentation medium should comprise at least 25 wt. % of a plant extract containing fermentable carbohydrates at the beginning of the process, and that the fermentation broth at the end of fermentation should contain at the most 9.5 wt. % magnesium lactate.

While the present disclosure enables the production of high-purity magnesium L-lactate from homogenous biomass-based feed streams, advantageously, it also enables the production of high-purity magnesium L-lactate from more complex feedstocks, such as non-homogeneous mixed food wastes from municipal, industrial, and commercial origin containing high contents of soluble and insoluble impurities. Additionally, the known methods are not suitable for fermentation broths derived from complex feedstocks having a wide range of possible initial fermentable carbohydrate concentrations, resulting in fermentation broths having a wide range of lactate concentrations. Surprisingly, the present inventors have found a simple and economic process for obtaining high purity magnesium L-lactate crystals that can be derived from a non-homogeneous fermentation broth obtained from complex feedstocks comprising a wide range of initial fermentable carbohydrate concentrations. Therefore, the present process is not confined or limited by the origin of the initial organic feedstock, or by the amount of fermentable carbohydrates in the initial organic feedstock.

The present disclosure advantageously enables the production of high-purity magnesium L-lactate crystals from heterogenous feedstocks with high yield and enantiomeric separation. Surprisingly, it is now disclosed for the first time that high purity magnesium L-lactate crystals can be obtained from decomposed organic waste containing endogenous D-lactic acid. Typically, organic waste comprises endogenous D-lactic acid, L-lactic acid or both L- and D- lactic acid, originating, for example, from natural fermentation processes, e.g., in dairy products. The present disclosure advantageously enables the production of high purity magnesium L-lactate crystals from decomposed organic waste containing endogenous D-lactic acid of up to 10wt.%.

According to the principles disclosed herein, after obtaining a clarified dispersion of decomposed organic waste or fermentation broth from which insoluble impurities have been removed, the concentration of lactate ions is optionally increased to a range of about 100 to about 150 g/L, including each value within the specified range. Preferably, lactate ions are concentrated to a concentration of about 100 to about 130 g/L, including each value within the specified range. Exemplary concentrations of the lactate after concentration include, but are not limited to, about 100, about 105, about 110, about 115, about 120, about 125, about 130, about 135, about 140, about 145, or about 150 g/L. Advantageously, a much lower lactate concentration of about 100 to about 150 g/L can be utilized as the initial concentration prior to crystallization thereby reducing costs and increasing efficacy. The process of lactate concentration is performed at elevated temperatures of about 50°C to about 90°C and the application of a vacuum to a pressure of about 80 to about 300 mbar, including each value within the specified ranges.

Thereafter, forming seed magnesium L-lactate crystals by mixing the clarified or concentrated dispersion or broth at elevated temperatures of about 50°C to about 90°C and the application of a vacuum to a pressure of about 80 to about 300 mbar, including each value within the specified ranges, is performed. Typically, the dispersion or broth is mixed using a mixer or homogenizer at a speed ranging from about 50 to about 300 rounds per minute (RPM), including each value within the specified range. Exemplary mixing speeds include, but are not limited to, about 50 RPM, about 75 RPM, about 100 RPM, about 125 RPM, about 150 RPM, about 175 RPM, about 200 RPM, about 225 RPM, about 250 RPM, about 275 RPM, or about 300 RPM. According to certain examples, the mixing is performed for at least 1 hour, at least 2 hours, at least 3 hours, at least 4 hours, at least 5 hours, at least 6 hours, or more. It is contemplated that the process disclosed herein may further comprise the addition of exogenous magnesium L-lactate seeds to facilitate the crystallization process.

Following the formation of seed magnesium L-lactate crystals, removal of about 70% to about 90% of water from the suspension is performed at elevated temperatures of about 50°C to about 90°C and the application of a vacuum to a pressure of about 80 to about 300 mbar, including each value within the specified ranges. Typically, at least about 70%, about 75%, about 80%, about 85%, or about 90% of water is removed at this stage.

Throughout these steps, the temperature is about 50°C, about 55°C, about 60°C, about 65°C, about 70°C, about 75°C, about 80°C, about 85°C, or about 90°C. The application of vacuum can be performed as is known in the art using e.g. a rotatory evaporator to a pressure of about 80, about 85, about 90, about 95, about 100, about 105, about 110, about 115, about 120, about 125, about 130, about 135, about 140, about 145, about 150, about 155, about 160, about 165, about 170, about 175, about 180, about 185, about 190, about 195, about 200, about 205, about 210, about 215, about 220, about 225, about 230, about 235, about 240, about 245, about 250, about 255, about 260, about 265, about 270, about 275, about 280, about 285, about 290, about 295, about 300, about 305, about 310, about 315, about 320, about 325, about 330, about 335, about 340, about 345, or about 350 mbar. Alternatively, throughout these steps, the temperature is about 100°C and no vacuum is applied.

According to certain examples, the rate of evaporation is in the range of about 2 to about 5% weight loss per hour, including each value within the specified range. Without being bound by any theory or mechanism of action, at this evaporation rate, the obtained magnesium L-lactate crystals exert the best filterability, overall purity and enantiomeric purity.

The obtained magnesium L-lactate crystals are then collected and separated from the remaining mother liquor. As used herein, the term "magnesium L-lactate" refers to the magnesium salt of hydroxycarboxylic acid (CH₃CH(OH)CO₂H) having the formula Mg(LA)₂. The term "magnesium L-lactate" refers to any solvate and/or polymorph of Mg(LA)₂ including, but not limited to, Mg(LA)₂ dihydrate. The term "mother liquor" as used herein, refers to the liquid remaining after the crystallization of the magnesium L-lactate crystals. In some examples, the obtained magnesium L-lactate crystals are separated from the remaining liquid by a method selected from microfiltration, nanofiltration, centrifugation, or another method known in the art. In some examples, the process disclosed herein further comprises a washing and/or purifying step, comprising washing and/or purifying the obtained magnesium L-lactate crystals. Washing can be performed using an organic solvent or an aqueous solution. In some examples, the organic solvent comprises one of more of ethanol, propanol, isobutanol, cyclohexane, acetone, ethyl acetate and combinations thereof. The aqueous solution comprises water. It is now disclosed for the first time that washing of the magnesium L-lactate crystals with water further improves the enantiomeric purity. Without being bound by any theory or mechanism of action, it is contemplated that the magnesium D-lactate crystals adhere to the surface of the magnesium L-lactate crystals thereby being washed by water to yield better enantiomeric purity. Accordingly, the present disclosure further provides a method of increasing the enantiomeric purity of magnesium L-lactate crystals, the method comprising washing magnesium L-lactate crystals obtained from crystallization of magnesium L-lactate at elevated temperature and reduced pressure with an aqueous solution. In some examples, the method comprises reducing the percentage of magnesium D-lactate in the magnesium L-lactate crystals by at least 50% (w/w). Preferably, washing is performed at room temperatures, for example at about 10°C, about 15°C, about 20°C, about 25°C, or about 30°C.

In some examples, the washing and/or purifying step further comprises at least one of a polishing step, extraction, microfiltration, nanofiltration, active carbon treatment, distillation, and grinding. In further examples, the process of the present disclosure further comprises a step of drying to reach a desirable % Loss on Drying. Exemplary LOD values include, but are not limited to, about 10% to about 20%, including each value within the specified range.

According to the principles disclosed herein, the process disclosed herein may further comprise solubilizing the collected magnesium L-lactate crystals in a suitable solvent and re-crystalizing them at elevated temperatures of about 50°C to about 90°C and the application of a vacuum to a pressure of about 80 to about 300 mbar, including each value within the specified ranges, as detailed above. This solubilization and recrystallization can be performed in additional cycles, for example at least one additional cycle, at least 2, at least 3, at least 4, at least 5, at least 6, or at least 10 additional cycles as needed to obtain the required purity of the crystals. However, it is to be understood that due to the improved purity of the obtained magnesium L-lactate crystals, a single cycle as disclosed herein suffices.

The recovery of the resulting magnesium L-lactate crystals is at least 90%, at least 91%, at least 92%, at least 93%, at least 94%, at least 95% or more. The magnesium L-lactate crystals obtained by the process disclosed herein may further be acidified to lactic acid for subsequent reuse.

In some examples, the resulting magnesium L-lactate crystals exert high purity of at least about 85 wt%, about 90%, about 92%, about 94 wt%, about 95 wt%, about 96 wt%, about 97 wt%, about 98 wt%, or about 99 wt%. In accordance with these examples, the magnesium L-lactate crystals comprise less than about 15 wt%, for example about 10 wt%, about 9 wt%, about 8 wt%, about 7 wt%, about 6 wt%, about 5 wt%, about 4 wt%, about 3 wt%, about 2 wt%, about 1 wt% or less of impurities.

In further examples, the resulting magnesium L-lactate crystals comprise less than 3%, for example about 2.9%, about 2.8%, about 2.7%, about 2.6%, about 2.5%, about 2.4%, about 2.3%, about 2.2%, about 2.1%, about 2.0%, about 1.9%, about 1.8%, about 1.7%, about 1.6%, about 1.5%, about 1.4%, about 1.3%, about 1.2%, about 1.1%, about 1% or less magnesium D-lactate. Thus, the process disclosed herein also provides enantiomeric enrichment for enriching L-lactate enantiomer from an enantiomeric mixture of D- and L-lactate monomers. This enrichment is particularly beneficial for reuse of lactic acid. The enrichment of L-lactate enantiomer by the process disclosed herein is at least 1%, 2%, 3%, 4%, 5%, 6%, 7%, 8%, 9%, 10% or even more of the initial L-lactate content. For example, for an initial enantiomeric mixture containing 90% L-lactate and 10% D-lactate, a 10% enrichment results in magnesium lactate salt containing 99% L-lactate and 1% D-lactate. Within the scope of the present disclosure is the reduction in D-lactate content by the process disclosed herein by at least 10%, 15%, 20%, 25%, 30%, 35%, 40%, 45%, 50%, 55%, 60%, 65%, 70%, 75%, 80%, 85%, 90%, 95%, or even 100% of the initial D-lactate content. For example, for an initial enantiomeric mixture containing 90% L-lactate and 10% D-lactate, a 50% reduction in D-lactate content results in magnesium lactate salt containing 95% L-lactate and 5% D-lactate.

In various examples, the resulting magnesium L-lactate crystals are characterized by a median particle size of less than 75 µm. In other examples, the resulting magnesium L-lactate crystals are characterized by a median particle size of more than 75 µm. The term "particle size" as used herein refers to the length of the particle (i.e. crystal) in the shortest dimension thereof. The particles have a shape selected from spherical, non-spherical, rectangular, flake, platelet, spongiform, and combinations thereof. Preferably, the resulting magnesium L-lactate crystals are characterized by a size distribution which can be a unimodal size distribution, a bimodal size distribution or a trimodal size distribution with a median particle size in the range of about 20 to about 100 µm, or about 100 to about 300 µm, including each value within the specified ranges. The term "median" or interchangeably "d₅₀" as used herein refers to a particle size in which the volume based cumulative distribution percentage reaches 50%. In other words, the median particle size represents a value where half the particles have diameters smaller than this value and half the particles have diameters larger than this value. Thus, the median particle size of the resulting magnesium L-lactate crystals is typically about 25, about 50, about 75, about 100, about 125, about 150, about 175, about 200, about 225, about 250, about 275, or about 300 µm.

While the processes disclosed herein are primarily contemplated for producing high-purity magnesium L-lactate crystals, the same processes may be likewise employed for the producing high-purity magnesium D-lactate crystals.

Thus, according to certain examples, there is provided a process for the formation of high-purity magnesium D-lactate crystals from decomposed organic waste, the process comprising the steps of:
a. providing a clarified dispersion of decomposed organic waste comprising a lactate salt in a concentration of about 50 to about 110 g/L;
b. optionally concentrating the clarified dispersion of step (a) to a lactate salt concentration of about 100 to about 150 g/L;
c. mixing the clarified dispersion of step (a) or the concentrated clarified dispersion of step (b) to obtain a suspension comprising seed magnesium D-lactate crystals;
d. removing from about 70% to about 90% of water from the suspension of step (c) to obtain magnesium D-lactate crystals; and
e. collecting the magnesium D-lactate crystals obtained in step (d),
wherein steps (b) through (d) are performed at elevated temperature in the range of about 50°C to about 90°C and the application of a vacuum to a pressure of about 80 to about 300 mbar, including each value within the specified ranges.

According to other examples, there is provided a process for enriching D-lactate enantiomer from an enantiomeric mixture derived from decomposed organic waste, the process comprising the steps of:
a. providing a clarified dispersion of decomposed organic waste comprising a lactate salt comprising an enantiomeric mixture of D- and L-lactate in a concentration of about 50 to about 110 g/L;
b. optionally concentrating the clarified dispersion of step (a) to a lactate salt concentration of about 100 to about 150 g/L;
c. mixing the clarified dispersion of step (a) or the concentrated clarified dispersion of step (b) to obtain a suspension comprising seed magnesium lactate crystals;
d. removing from about 70% to about 90% of water from the suspension of step (c) to obtain magnesium D-lactate crystals with enriched enantiomeric purity; and
e. collecting the magnesium D-lactate crystals obtained in step (d),
wherein steps (b) through (d) are performed at elevated temperature in the range of about 50°C to about 90°C and the application of a vacuum to a pressure of about 80 to about 300 mbar, including each value within the specified ranges.

The term "about" as used herein refers to ±10% of a specified value.

Throughout the description and claims, the words "comprise" and "contain" and variations of the words, for example "comprising" and "comprises", mean "including but not limited to", and are not intended to (and do not) exclude other moieties, additives, components, integers or steps.

As used herein, the singular forms "a", "an", and "the" include plural forms unless the context clearly dictates otherwise. Thus, for example, reference to "a solvent" includes combinations of solvents as is known in the art.

As used herein, the term "and" or the term "or" include "and/or" unless the context clearly dictates otherwise.

Various embodiments and aspects of the present invention as delineated hereinabove and as claimed in the claims section below find experimental support in the following examples.

### EXAMPLES

### Example 1.

Fermentation of mixed food waste feedstock was performed using magnesium hydroxide as an alkaline agent which was added to maintain a pH in the range of 5-7. The lactate containing fermentation broth was centrifuged and filtered to produce a clear supernatant with a lactate concentration of 75 g/L. The supernatant was maintained at 60°C and a vacuum of 180 mbar to a lactate concentration of 120 g/L. The concentrated supernatant was stirred at 200 RPM for 4 hours under the same conditions to allow seed crystals formation, then concentrated again, removing 80% of the water. The concentrate was cooled to 25°C and filtered using a Buchner funnel equipped with Whatman 3 filtration paper. The obtained crystals were washed with acetone and dried at 80°C. The yield of production was 70% w/w with an assay of 97.4%. While the D/L-lactate ratio in the supernatant was initially 3.7%/96.3%, the D/L-lactate ratio of the mother-liquor was 8.6%/91.4%, and the obtained D/L-lactate ratio in the magnesium lactate crystals was 1.2%/98.8%. Surprisingly, washing of the magnesium lactate crystals with water further improved the enantiomeric purity. Without being bound by any theory or mechanism of action, it is contemplated that the D-lactate crystals adhere to the surface of the L-lactate crystals thereby being washed by water to yield better enantiomeric purity.

### Example 2.

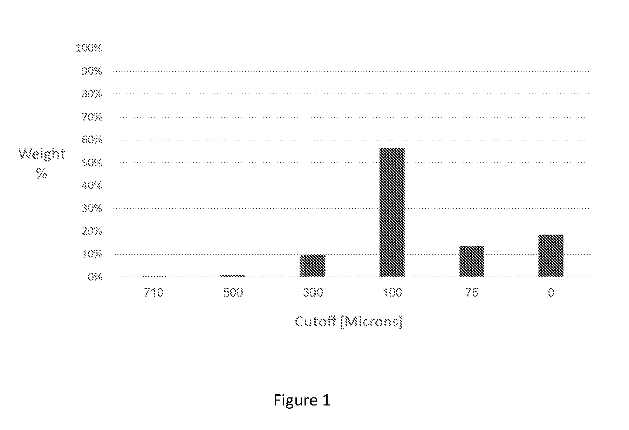

Magnesium L-lactate crystals produced as described in Example 1 were assessed for their size distribution using a series of sieves with the following size cutoffs: 75 µm, 100 µm, 300 µm, and 710 µm. The obtained size distribution profile is presented in Figure 1 and Table 1.

**Table 1.**

| **Sieve cutoff [µm]** | **Weight %** |
|---|---|
| 710 | 0.16 |
| 500 | 1.13 |
| 300 | 9.91 |
| 100 | 56.41 |
| 75 | 13.71 |
| 0 | 18.68 |

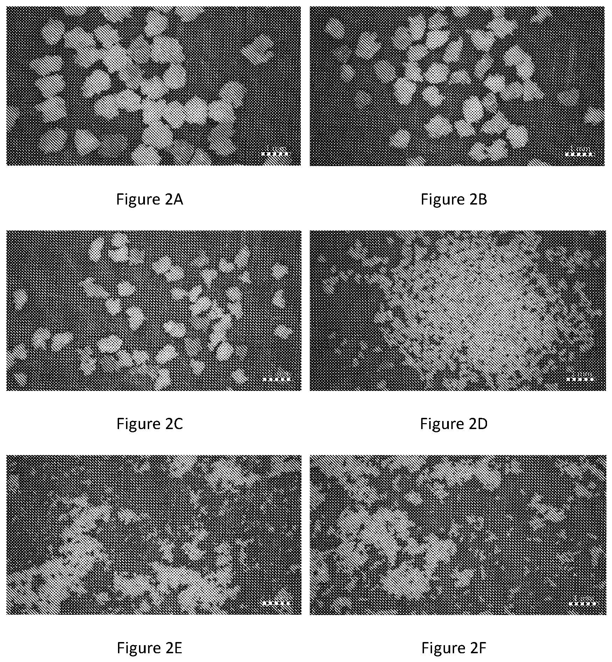

The crystals were observed in a light microscope. Figures 2A-2F show that the crystals exhibited a relatively uniform shape with characteristic crystalline facets.

### Example 3.

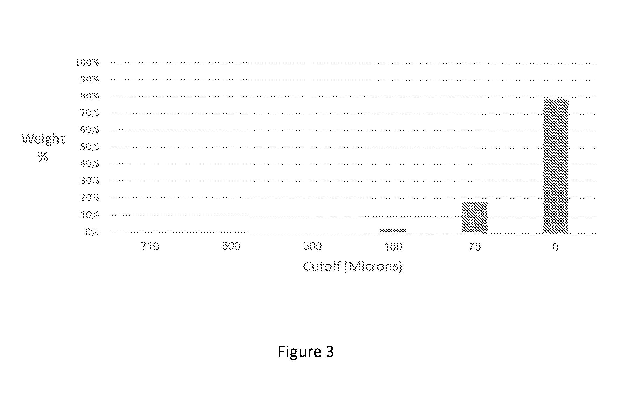

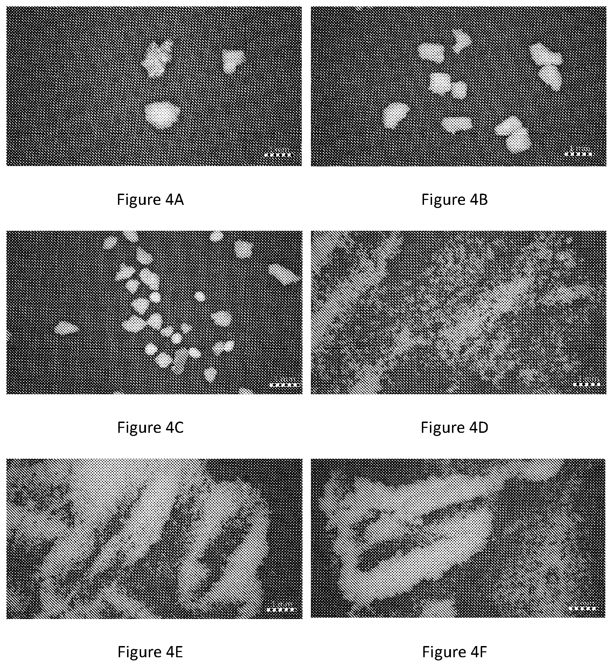

The crystals produced as described in Example 1 and characterized as described in Example 2 were compared to crystals obtained by a process in which a clarified fermentation broth with a lactate concentration of 98 g/L was concentrated to a lactate concentration of 215 g/L at 60°C. The concentrate was cooled to 20°C at a rate of 2°C/min and the crystals were filtered and washed with cold water (5°C). The size distribution of the crystals obtained by the process described in this example is presented in Figure 3 and Table 2 and the light microscopy images of the crystals are shown in Figures 4A-4F.

**Table 2.**

| **Sieve cutoff [µm]** | **Weight %** |
|---|---|
| 710 | 0.02 |
| 500 | 0.05 |
| 300 | 0.03 |
| 100 | 2.26 |
| 75 | 18.26 |
| 0 | 79.37 |

### Example 4.

Fermentation of mixed food waste feedstock was performed using magnesium hydroxide as an alkaline agent which was added to maintain a pH in the range of 5-7. The lactate containing fermentation broth was centrifuged and filtered to produce a clear supernatant with a lactate concentration of 78 g/L. The supernatant was maintained at 60°C and a vacuum of 200 mbar to a lactate concentration of 122 g/L. The concentrated supernatant was stirred at 200 RPM for 4 hours under the same conditions to allow seed crystals formation, then concentrated again, removing 80% of the water. The concentrate was cooled to 25°C and filtered using a Buchner funnel equipped with Whatman 3 filtration paper. The obtained crystals were washed with acetone and water, and dried at 80°C. The yield of production was 74% w/w with an assay of 96.0%. While the D/L-lactate ratio in the supernatant was initially 3.1%/96.9%, the D/L-lactate ratio of the mother liquor was 8.3%/91.7%, and the obtained D/L-lactate ratio in the magnesium lactate crystals was 1.6%/98.4%. The obtained magnesium lactate crystals were redissolved to a final concentration of 110 g/L, the solution was treated with activated carbon to remove colored impurities and filtered. The clear solution was stirred at 200 RPM for 6 hours at 60°C to allow seed crystals formation, then concentrated again, removing 80% of the water. The concentrate was cooled to 30°C and filtered using a Buchner funnel equipped with Whatman 3 filtration paper. The obtained crystals were washed with acetone and water, and dried at 70°C. The yield of production was 66% w/w with an assay of 98.4%. While the D/L-lactate ratio in the re-crystallization solution was initially 1.6%/98.4%, the D/L-lactate ratio of the mother liquor was 5.3%/94.7%, and the obtained D/L-lactate ratio in the magnesium lactate crystals was 0.8%/99.2%.

### Example 5.

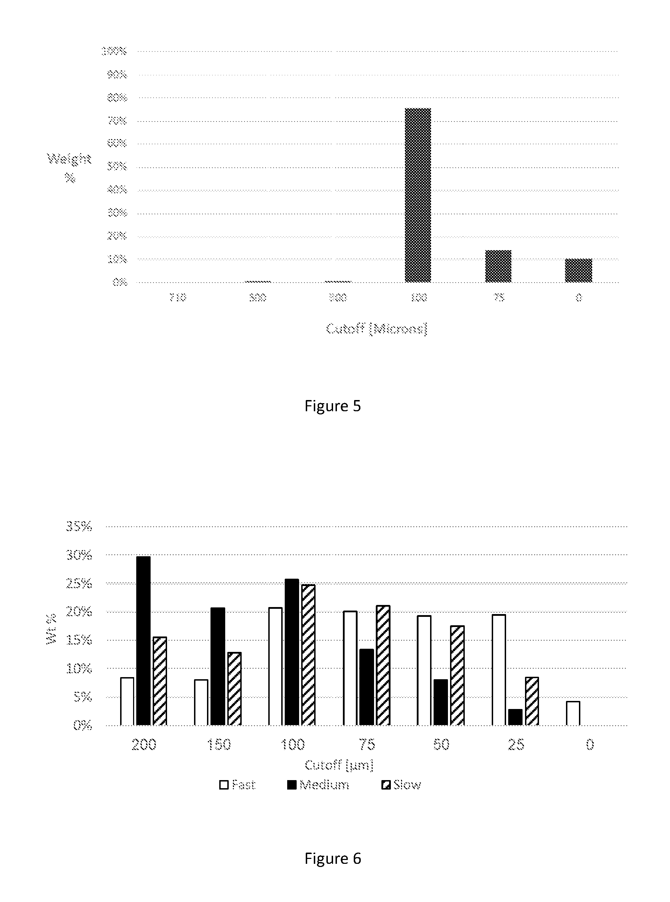

Magnesium L-lactate crystals produced as described in Example 4 were assessed for their size distribution as described in Example 2. The obtained size distribution profile is presented in Figure 5 and Table 3.

**Table 3.**

| **Sieve cutoff [µm]** | **Weight %** |
|---|---|
| 710 | 0.0 |
| 500 | 1.01 |
| 300 | 0.37 |
| 100 | 75.46 |
| 75 | 13.84 |
| 0 | 10.32 |

As evident from these results, the crystals obtained by the process of the present disclosure as described in Examples 1 and 4 were much larger than those obtained by a cooling crystallization process as described in Example 3. Specifically, whereas the majority of the crystals obtained by the process of the disclosure were larger than 100 microns, those obtained by the cooling crystallization process were smaller than 75 microns. Also, the crystals obtained by the cooling crystallization process showed very little characteristic crystalline facets and were mostly in the form of aggregates.

### Example 6.

In order to examine the effect of evaporation rate on the obtained magnesium lactate crystal attributes, three crystallization experiments termed "slow", "medium", and "fast" were conducted at different crystallization conditions as detailed in Table 4. The experiments were performed on clarified broth derived from a lactic acid fermentation experiment as described in Examples 1 and 4.

**Table 4.**

| | **Reactor size** | **Temperature** | **Pressure** | **Stirring rate** | **Avg. evap. rate** | **evaporation time** |
|---|---|---|---|---|---|---|
| | [L] | [°C] | [mbar] | [RPM] | [wt%/h] | [h] |
| **Slow** | 0.5 | 70 | 315 | 300 | 0.7% | 51.5 |
| **Medium** | 0.5 | 70 | 280 | 300 | 4.3% | 9 |
| **Fast** | 0.5 | 70 | 250 | 300 | 12.6% | 3.5 |

Four different parameters were assessed as follows: a) the cake moisture-measured after the initial filtration and before the aqueous wash. The cake moisture demonstrates filterability whereby a lower value indicates that more mother liquor was removed in the filtration; b) the crystal purity- measured using HPLC; c) the yield-(corrected according to crystal purity); and d) the %D-lactate- measured using HPLC. The results are presented in Table 5.

**Table 5.**

| | **Cake moisture** | **Yield** | **Crystal purity** | **%D-lactate** |
|---|---|---|---|---|
| **Slow** | 18% | 69.0% | 95.9% | 3.1% |
| **Medium** | 13% | 58.0% | 96.6% | 2.3% |
| **Fast** | 17% | 46.0% | 93.5% | 2.5% |

The medium evaporation rate showed the best results in terms of filterability with 4-5% less water than the slow or fast evaporation rates. This rate also yielded crystals with the highest purity and the lowest %D-lactate. A trend of reduction in total yield of the crystallization at high evaporation rates were detected.

Crystals obtained from the experiments were also assessed by inductively - coupled plasma (ICP) elemental analysis. The results are presented in Table 6.

**Table 6.**

| **Element** | **Slow** | **Medium** | **Fast** |
|---|---|---|---|
| Al | 8.33 | 1.69 | 3.32 |
| Ca | 1065 | 368 | 495 |
| Cl | 1099.04 | 574.16 | 808.85 |
| K | 646 | 269 | 377 |
| Mn | 7.76 | 8.31 | 8.62 |
| Na | 591 | 282 | 372 |
| P | 391 | 90 | 128 |
| S | 98.0 | 40.2 | 54.3 |
| Zn | 5.7 | 4.4 | 3.8 |

These results indicate that the medium evaporation rate crystallization was superior in removing elemental impurities during crystallization. In particular, calcium as well as chloride, potassium, sodium and phosphorous concentrations were reduced significantly by the medium-rate crystallization as compared to crystallizations at slow or fast evaporation rates. The size distribution of the crystals obtained were also assessed using sieves with different cutoffs. The results indicate that the medium-rate crystallization formed the largest crystals, with >75% of the crystals being larger than 100 µm. The results are shown in Figure 6. It is noted, however, that size distribution based on sieving is less accurate due to the crystals' tendency to form clusters.

Taken together, these results show that medium rate crystallization produced the highest quality of magnesium lactate crystals.

### Example 7.

Lactic acid fermentation broth was centrifuged and filtered to produce 1 kg of a clear supernatant with 7% lactate. The supernatant was concentrated using a rotatory evaporator to 12% lactate, 42 wt% were removed. The resulting concentrate was transferred to a 0.5L reactor pre-heated to 70°C and stirred at 300 RPM. The supernatant was reduced in vacuo (315, 280 or 250 mbar) at a rate of 7, 65 or 135 g/h, respectively. After removal of 80 wt%, the crystals were harvested and filtered using a sintered glass funnel. The obtained crystals were washed with cold water and dried at 70°C. As exemplified in Example 6, the medium crystallization rate in which evaporation occurs during the course of 8-16h with an evaporation rate of 2.4-4.8wt% per hour provided the best results.

### Example 8.

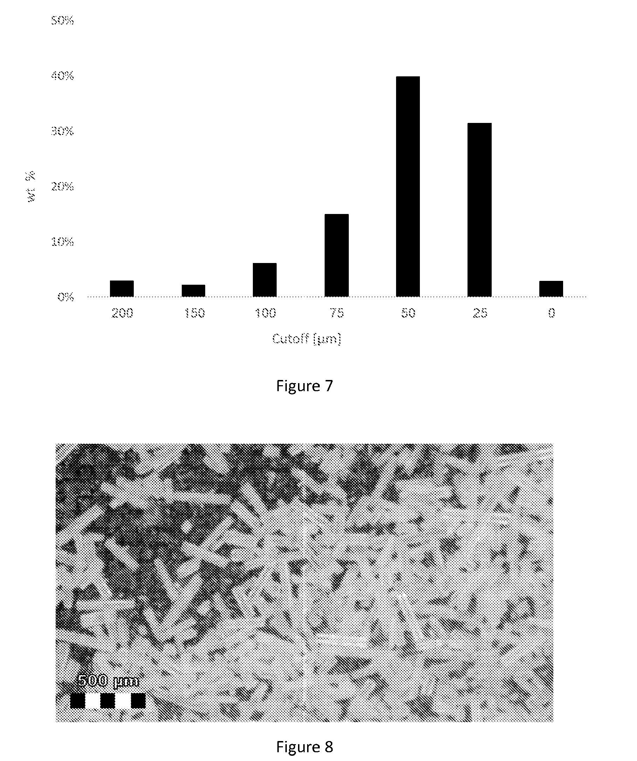

Fed batch crystallization with constant mass was performed. Lactic acid fermentation broth was centrifuged and filtered to produce 2 kg of a clear supernatant with 7% lactic acid. 20% of the supernatant (0.4 kg) were added to a 0.5L reactor pre-heated to 85°C and stirred at 300 RPM. The remaining 80% (1.6 kg) were added to a separate vessel heated to 60°C and connected to the reactor with a peristaltic pump. The supernatant was reduced in vacuo (280 mbar) at a rate of 80 g/h. During evaporation, supernatant was added at the same rate. Internal temperature during the crystallization was maintained at 70°C. After 20h, all supernatant was added following which crystals were harvested and filtered using a sintered glass funnel. The obtained crystals were washed with cold water and dried at 70°C. Yield: 57%, Assay: 98.6%, %D-lactate: 3.8%, Original %D-lactate: 7.0%. The crystals were characterized for their size distribution through sieving. The results are shown in Figure 7. The crystals were observed in a light microscope. Figure 8 shows an image of crystals that remained on top of a 50µm sieve.

### Example 9.

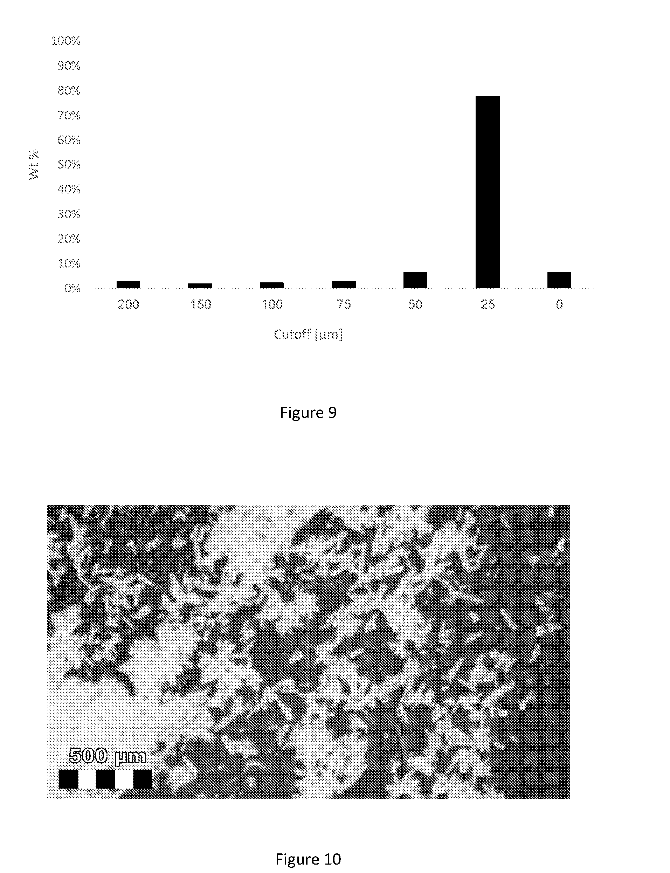

Fed batch crystallization with constant concentration was performed. Lactic acid fermentation broth was centrifuged and filtered to produce 2 kg of a clear supernatant with a lactic acid concentration of 70 g/L. 50% of the supernatant (1 kg) were concentrated using a rotatory evaporator to a lactic acid concentration of 120 g/L. The resulting concentrate was transferred to a 0.5L reactor pre-heated to 80°C and stirred at 300 RPM. The remaining 50% (1 kg) of supernatant were added to a separate vessel heated to 60°C and connected to the reactor with a peristaltic pump. The concentrate was reduced in vacuo (180 mbar) at a rate of 100 g/h until reaching a total concentration factor of 80% (200 g). Internal temperature during the crystallization was maintained at 58°C. The supernatant was then added at a rate of 80 g/h, while maintaining an 80% evaporation ratio. After 12.5h, all supernatant was added. The reactor was then left to stir at 60°C at atmospheric pressure for 7h. Crystals were harvested and filtered using a sintered glass funnel. The obtained crystals were washed with cold water and dried at 70°C. Yield: 43%, Assay: 96.3%. The crystals were characterized for their size distribution through sieving. The results are shown in Figure 9. The crystals were observed in a light microscope. Figure 10 shows an image of crystals that remained on top of a 25µm sieve.

### Example 10.

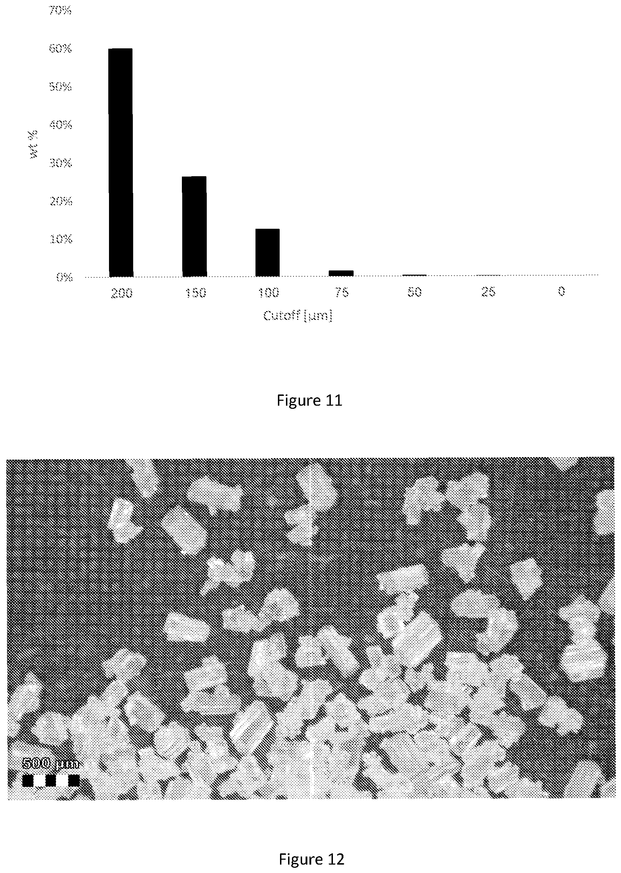

Magnesium lactate dihydrate was obtained from decomposition of polylactic acid (PLA 4032D) using sodium hydroxide to obtain sodium lactate slurry. The sodium ions were replaced with magnesium ions by adding magnesium sulfate as described in WO 2021/165964. Magnesium lactate dihydrate was then added to a three-necked round bottom flask equipped with a condenser, and dissolved in DW at 100°C. Any undissolved impurities were filtered off using a sintered glass funnel. The resulting 580 g of clear solution (10.7% lactic acid) were transferred to a 0.5 L reactor pre-heated to 100°C. The solution was allowed to boil and water evaporated during 20h, with a total of 320 g being removed. Crystals were then harvested and filtered using a sintered glass funnel. The obtained crystals were dried at 70°C. Yield: 57.5%, Assay: 97.0%, %D-lactate: 0.5%, Original crystals assay: 86.4%; Original %D-lactate: 1.5%. The crystals were characterized for their size distribution through sieving. The results are shown in Figure 11. The crystals were observed in a light microscope. Figure 12 shows an image of crystals that remained on top of a 200µm sieve.

### Example 11.

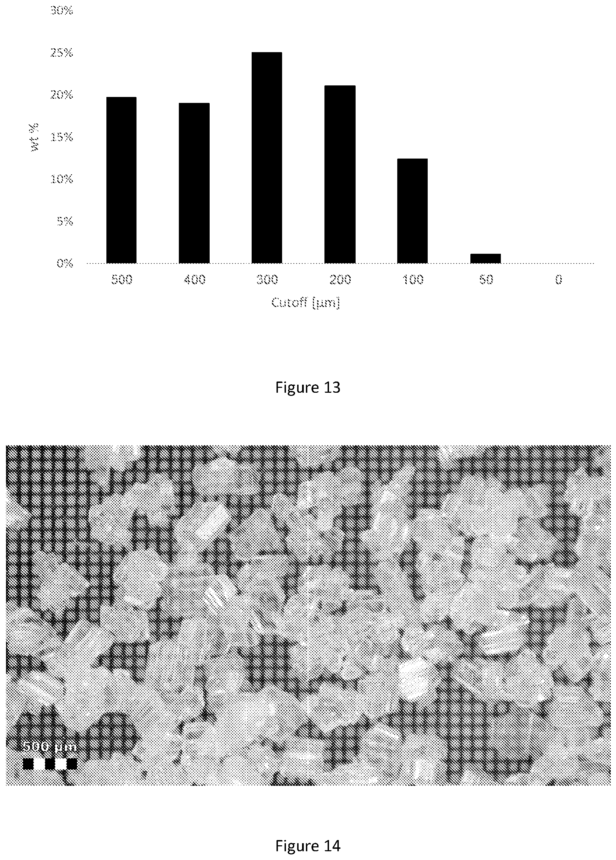

Magnesium lactate dihydrate was obtained from decomposition of polylactic acid (tableware waste) using sodium hydroxide to obtain sodium lactate slurry. The sodium ions were replaced with magnesium ions by adding magnesium sulfate as described in WO 2021/165964. Magnesium lactate dihydrate was added to a three-necked round bottom flask equipped with a condenser, and dissolved in DW at 100°C. Any undissolved impurities were filtered off using a sintered glass funnel. The resulting 500 g of clear solution (8.8% LA) were transferred to a 0.5 L reactor pre-heated to 100°C. The solution was allowed to boil and water evaporated during 29h with a total of 336 g being removed. Crystals were then harvested and filtered using a sintered glass funnel. The obtained crystals were washed with cold water and dried at 70°C. Yield: 70.7%, Assay: 98.0%, %D-lactate: 0.6%, Original crystals assay: 82.1%, Original %D-lactate: 3.8%. The crystals were characterized for their size distribution through sieving. The results are shown in Figure 13. The crystals were observed in a light microscope. Figure 14 shows an image of crystals that remained on top of a 300µm sieve.

### Comparative Example

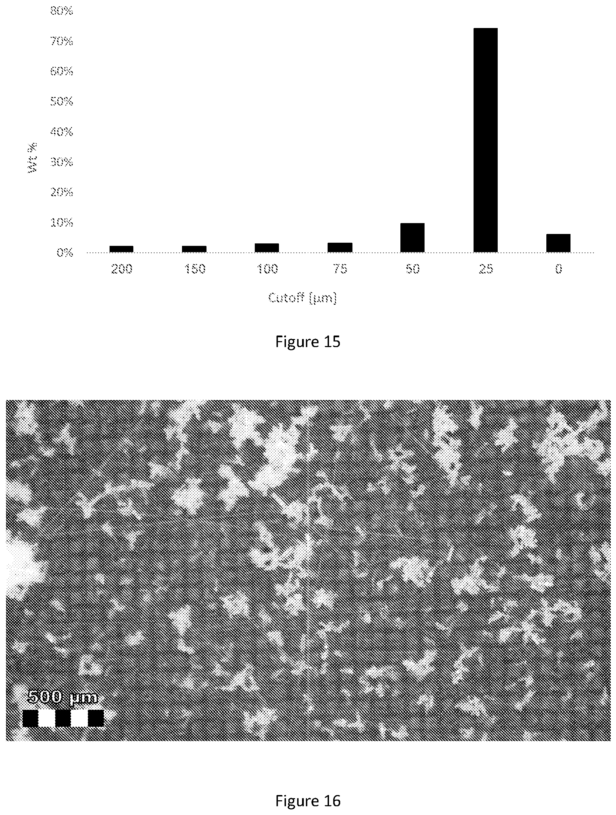

Magnesium lactate dihydrate was obtained from lactic acid fermentation broth using evaporative crystallization at 30°C. In particular, lactic acid (LA) fermentation broth was centrifuged and filtered to produce 1 kg of a clear supernatant with 7% LA. The supernatant was maintained at 30°C with the application of vacuum at 30-40 mbar until a lactic acid concentration of 12% was reached. The concentrated supernatant was transferred to a 0.5L reactor pre-heated to 30°C and stirred at 300 RPM, then concentrated again in vacuo (35-40 mbar), removing 77% of the water. The crystals were then harvested and filtered using a sintered glass funnel. Original %D-lactate: 6.8%, %D-lactate in mother-liquor: 6.0%, Yield: 88%, Assay: 84.6%. The obtained crystals were washed with cold water and dried at 70°C. Yield was reduced to 54% and assay was increased to 99.8%. Thus, contrary to crystallizations of magnesium L-lactate at temperatures in the range of about 50-100°C according to embodiments of the present disclosure, the crystallization at 30°C did not result in an increase in the D-lactate content in the mother liquor as compared to the initial value. The D-lactate content in the mother liquor rather decreased resulting in crystals having the same ratio of D-lactate to L-lactate as the original filtered broth. Also, the obtained crystals were very small and are contemplated to be less suitable for new PLA production (Figures 15-16).

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. The scope of the invention shall be defined by the appended claims, solely.

## Claims

1. A process for the formation of high-purity magnesium L-lactate crystals from decomposed organic waste and/or enriching L-lactate enantiomer from an enantiomeric mixture of D- and L-lactate derived from decomposed organic waste, the process comprising the steps of:
a. providing a clarified dispersion of decomposed organic waste comprising a lactate salt in a concentration of about 50 to about 110 g/L;
b. optionally concentrating the clarified dispersion of step (a) to a lactate salt concentration of about 100 to about 150 g/L;
c. mixing the clarified dispersion of step (a) or the concentrated clarified dispersion of step (b) to obtain a suspension comprising seed magnesium L-lactate crystals;
d. removing from about 70% to about 90% of water from the suspension of step (c) to obtain magnesium L-lactate crystals at high purity and/or enriched enantiomeric purity; and
e. collecting the magnesium L-lactate crystals obtained in step (d),
**characterized in that** steps (b) through (d) are performed at elevated temperature in the range of about 50°C to about 90°C and the application of a vacuum to a pressure of about 80 to about 300 mbar.

2. The process according to claim 1, wherein the clarified dispersion of decomposed organic waste comprises decomposed organic waste from which impurities have been removed using at least one of filtration, centrifugation, flotation, sedimentation, coagulation, flocculation, and decantation; or wherein the organic waste is selected from food waste, municipal food waste, residential food waste, agricultural waste, industrial food waste from food processing facilities, commercial food waste, and a mixture or combination thereof.

3. The process according to claim 1, wherein the decomposed organic waste comprises a fermentation broth, preferably wherein the fermentation broth is obtained from a fermentation process of a carbohydrate source or wherein the fermentation broth is obtained from a fermentation process of an organic waste feedstock.

4. The process according to any one of claims 1 to 3, wherein step (b) is performed to a lactate salt concentration of about 100 to about 130 g/L.

5. The process according to any one of claims 1 to 4, wherein the mixing in step (c) is performed at a speed of about 50 to about 300 rounds per minute (RPM); or wherein the mixing in step (c) is performed for at least 1 hour.

6. The process according to any one of claims 1 to 5, wherein steps (b) through (d) are performed at elevated temperature in the range of about 50°C to about 80°C; or wherein steps (b) through (d) are performed with the application of a vacuum to a pressure of about 150 to about 250 mbar.

7. The process according to any one of claims 1 to 6, wherein step (d) is performed at an evaporation rate of loss of about 2 to about 5 wt% of the total weight of the suspension per hour; or wherein step (e) comprises filtration and/or centrifugation; or wherein step (e) is performed at room temperatures.

8. The process according to any one of claims 1 to 7, further comprising step (f) of washing the obtained magnesium L-lactate crystals, preferably wherein washing of the obtained magnesium L-lactate crystals is performed in a solvent selected from water, ethanol, propanol, isobutanol, cyclohexane, acetone, ethyl acetate, and a mixture or combination thereof.

9. The process according to any one of claims 1 to 8, further comprising step (g) of drying the magnesium L-lactate crystals to a Loss on Drying (LOD) % of about 10% to about 20%, preferably wherein drying is performed at elevated temperatures of about 50°C to about 120°C.

10. The process according to any one of claims 1 to 9, wherein the obtained magnesium L-lactate crystals are solubilized and re-crystalized by reiterating steps (c) to (e); or wherein the obtained magnesium L-lactate crystals are **characterized by** a median size smaller than 75 µm; or wherein the obtained magnesium L-lactate crystals are **characterized by** a median size larger than 75 µm; or wherein the obtained magnesium L-lactate crystals are **characterized by** a size distribution comprising a median size in the range of about 100 to about 300 µm.

11. The process according to any one of claims 1 to 10, wherein the recovery of magnesium L-lactate crystals is at least 90%.

12. The process according to any one of claims 1 to 11, wherein the obtained magnesium L-lactate crystals comprise less than 3% magnesium D-lactate, preferably less than 2% magnesium D-lactate, more preferably less than 1.5% magnesium D-lactate, and most preferably less than 1% magnesium D-lactate.

## Patentansprüche

1. Verfahren zur Bildung von hochreinen Magnesium-L-Lactat-Kristallen aus zersetztem organischem Abfall und/oder zum Anreichern eines L-Lactat-Enantiomers aus einer Enantiomerenmischung von D- und L-Lactat, das aus zersetztem organischem Abfall stammt, wobei das Verfahren die folgenden Schritte umfasst:
a. Bereitstellen einer geklärten Dispersion von zersetztem organischem Abfall, umfassend ein Lactatsalz in einer Konzentration von etwa 50 bis etwa 110 g/l;
b. optional Konzentrieren der geklärten Dispersion von Schritt (a) auf eine LactatSalzkonzentration von etwa 100 bis etwa 150 g/l;
c. Mischen der geklärten Dispersion von Schritt (a) oder der konzentrierten geklärten Dispersion von Schritt (b), um eine Suspension, umfassend Magnesium-L-Lactat-Impfkristalle, zu erlangen;
d. Entfernen von etwa 70 % bis etwa 90 % Wasser aus der Suspension von Schritt (c), um Magnesium-L-Lactat-Kristalle mit hoher Reinheit und/oder angereicherter Enantiomerenreinheit zu erlangen; und
e. Sammeln der in Schritt (d) erlangten Magnesium-L-Lactat-Kristalle, **dadurch gekennzeichnet, dass** die Schritte (b) bis (d) bei erhöhter Temperatur in dem Bereich von etwa 50 °C bis etwa 90 °C und unter der Anwendung eines Vakuums mit einem Druck von etwa 80 bis etwa 300 mbar durchgeführt werden.

2. Verfahren nach Anspruch 1, wobei die geklärte Dispersion von zersetztem organischem Abfall zersetzten organischen Abfall umfasst, aus dem Verunreinigungen mithilfe mindestens eines von Filtration, Zentrifugation, Flotation, Sedimentation, Koagulation, Flockung und Dekantation entfernt wurden; oder wobei der organische Abfall ausgewählt ist aus Lebensmittelabfall, kommunalem Lebensmittelabfall, Haushaltslebensmittelabfall, landwirtschaftlichem Abfall, industriellem Lebensmittelabfall aus Lebensmittelverarbeitungsbetrieben, gewerblichem Lebensmittelabfall und einer Mischung oder Kombination davon.

3. Verfahren nach Anspruch 1, wobei der zersetzte organische Abfall eine Fermentationsbrühe umfasst, wobei die Fermentationsbrühe bevorzugt aus einem Fermentationsprozess einer Kohlenhydratquelle erlangt ist oder wobei die Fermentationsbrühe aus einem Fermentationsprozess eines organischen Abfallrohstoffs erlangt ist.

4. Verfahren nach einem der Ansprüche 1 bis 3, wobei Schritt (b) bis zu einer Lactatsalzkonzentration von etwa 100 bis etwa 130 g/l durchgeführt wird.

5. Verfahren nach einem der Ansprüche 1 bis 4, wobei das Mischen in Schritt (c) mit einer Geschwindigkeit von etwa 50 bis etwa 300 Umdrehungen pro Minute (U/min) durchgeführt wird; oder wobei das Mischen in Schritt (c) mindestens 1 Stunde lang durchgeführt wird.

6. Verfahren nach einem der Ansprüche 1 bis 5, wobei Schritte (b) bis (d) bei erhöhter Temperatur in dem Bereich von etwa 50 °C bis etwa 80 °C durchgeführt werden; oder wobei Schritte (b) bis (d) unter der Anwendung eines Vakuums mit einem Druck von etwa 150 bis etwa 250 mbar durchgeführt werden.

7. Verfahren nach einem der Ansprüche 1 bis 6, wobei Schritt (d) mit einer Verdunstungsrate von etwa 2 bis etwa 5 Gew.-% des Gesamtgewichts der Suspension pro Stunde durchgeführt wird; oder wobei Schritt (e) Filtration und/oder Zentrifugation umfasst; oder wobei Schritt (e) bei Raumtemperatur durchgeführt wird.

8. Verfahren nach einem der Ansprüche 1 bis 7, ferner umfassend Schritt (f) zum Waschen der erlangten Magnesium-L-Lactat-Kristalle, wobei das Waschen der erlangten Magnesium-L-Lactat-Kristalle bevorzugt in einem Lösungsmittel durchgeführt wird, das ausgewählt ist aus Wasser, Ethanol, Propanol, Isobutanol, Cyclohexan, Aceton, Ethylacetat und einer Mischung oder Kombination davon.

9. Verfahren nach einem der Ansprüche 1 bis 8, ferner umfassend Schritt (g) zum Trocknen der Magnesium-L-Lactat-Kristalle auf einen Trocknungsverlust-(LOD-)Prozentanteil von etwa 10 % bis etwa 20 %, wobei das Trocknen bevorzugt bei erhöhten Temperaturen von etwa 50 °C bis etwa 120 °C durchgeführt wird.

10. Verfahren nach einem der Ansprüche 1 bis 9, wobei die erlangten Magnesium-L-Lactat-Kristalle durch Wiederholen der Schritte (c) bis (e) aufgelöst und rekristallisiert werden; oder wobei die erlangten Magnesium-L-Lactat-Kristalle durch eine mittlere Größe von weniger als 75 µm gekennzeichnet sind; oder wobei die erlangten Magnesium-L-Lactat-Kristalle durch eine mittlere Größe von mehr als 75 µm gekennzeichnet sind; oder wobei die erlangten Magnesium-L-Lactat-Kristalle durch eine Größenverteilung, umfassend eine mittlere Größe in dem Bereich von etwa 100 bis etwa 300 µm, gekennzeichnet sind.

11. Verfahren nach einem der Ansprüche 1 bis 10, wobei die Gewinnung von Magnesium-L-Lactat-Kristallen mindestens 90 % ist.

12. Verfahren nach einem der Ansprüche 1 bis 11, wobei die erlangten Magnesium-L-Lactat-Kristalle weniger als 3 % Magnesium-D-Lactat, bevorzugt weniger als 2 % Magnesium-D-Lactat, mehr bevorzugt weniger als 1,5 % Magnesium-D-Lactat und am meisten bevorzugt weniger als 1 % Magnesium-D-Lactat umfassen.

## Revendications

1. Procédé de formation de cristaux de L-lactate de magnésium de haute pureté à partir de déchets organiques décomposés et/ou d'enrichissement de l'énantiomère L-lactate à partir d'un mélange énantiomérique de D- et L-lactate issu de déchets organiques décomposés, le procédé comprenant les étapes suivantes :
a. la fourniture d'une dispersion clarifiée de déchets organiques décomposés comprenant un sel de lactate dans une concentration d'environ 50 à environ 110 g/L ;
b. éventuellement la concentration de la dispersion clarifiée de l'étape (a) à une concentration en sel de lactate d'environ 100 à environ 150 g/L ;
c. le mélange de la dispersion clarifiée de l'étape (a) ou de la dispersion clarifiée concentrée de l'étape (b) pour obtenir une suspension comprenant des cristaux de semence de L-lactate de magnésium ;
d. le retrait d'environ 70 % à environ 90 % d'eau de la suspension de l'étape (c) pour obtenir des cristaux de L-lactate de magnésium de haute pureté et/ou de pureté énantiomérique enrichie ; et
e. la collecte des cristaux de L-lactate de magnésium obtenus à l'étape (d), **caractérisé en ce que** les étapes (b) à (d) sont réalisées à une température élevée comprise entre environ 50 °C et environ 90 °C et l'application d'un vide à une pression d'environ 80 à environ 300 mbar.

2. Procédé selon la revendication 1, dans lequel la dispersion clarifiée de déchets organiques décomposés comprend des déchets organiques décomposés dont les impuretés ont été éliminées à l'aide d'au moins l'une des méthodes suivantes : filtration, centrifugation, flottation, sédimentation, coagulation, floculation et décantation ; ou dans lequel les déchets organiques sont choisis parmi les déchets alimentaires, les déchets alimentaires municipaux, les déchets alimentaires résidentiels, les déchets agricoles, les déchets alimentaires industriels provenant d'installations de transformation des aliments, les déchets alimentaires commerciaux et un mélange ou une combinaison de ceux-ci.

3. Procédé selon la revendication 1, dans lequel les déchets organiques décomposés comprennent un bouillon de fermentation, de préférence dans lequel le bouillon de fermentation est obtenu à partir d'un procédé de fermentation d'une source de glucides ou dans lequel le bouillon de fermentation est obtenu à partir d'un procédé de fermentation d'une charge d'alimentation de déchets organiques.

4. Procédé selon l'une quelconque des revendications 1 à 3, dans lequel l'étape (b) est réalisée à une concentration en sel de lactate d'environ 100 à environ 130 g/L.

5. Procédé selon l'une quelconque des revendications 1 à 4, dans lequel le mélange à l'étape (c) est réalisé à une vitesse d'environ 50 à environ 300 tours par minute (RPM) ; ou dans lequel le mélange à l'étape (c) est réalisé pendant au moins 1 heure.

6. Procédé selon l'une quelconque des revendications 1 à 5, dans lequel les étapes (b) à (d) sont réalisées à une température élevée comprise entre environ 50 °C et environ 80 °C ; ou dans lequel les étapes (b) à (d) sont réalisées avec l'application d'un vide à une pression d'environ 150 à environ 250 mbar.

7. Procédé selon l'une quelconque des revendications 1 à 6, dans lequel l'étape (d) est réalisée à un taux d'évaporation de perte d'environ 2 à environ 5 % en poids du poids total de la suspension par heure ; ou dans lequel l'étape (e) comprend une filtration et/ou une centrifugation ; ou dans lequel l'étape (e) est réalisée à température ambiante.

8. Procédé selon l'une quelconque des revendications 1 à 7, comprenant également l'étape (f) de lavage des cristaux de L-lactate de magnésium obtenus, de préférence dans lequel le lavage des cristaux de L-lactate de magnésium obtenus est réalisé dans un solvant choisi parmi l'eau, l'éthanol, le propanol, l'isobutanol, le cyclohexane, l'acétone, l'acétate d'éthyle et un mélange ou une combinaison de ceux-ci.

9. Procédé selon l'une quelconque des revendications 1 à 8, comprenant également l'étape (g) de séchage des cristaux de L-lactate de magnésium à une perte au séchage (LOD) % d'environ 10 % à environ 20 %, de préférence dans lequel le séchage est réalisé à des températures élevées d'environ 50 °C à environ 120 °C.

10. Procédé selon l'une quelconque des revendications 1 à 9, dans lequel les cristaux de L-lactate de magnésium obtenus sont solubilisés et recristallisés en répétant les étapes (c) à (e) ; ou dans lequel les cristaux de L-lactate de magnésium obtenus sont **caractérisés par** une taille médiane inférieure à 75 µm ; ou dans lequel les cristaux de L-lactate de magnésium obtenus sont **caractérisés par** une taille médiane supérieure à 75 µm ; ou dans lequel les cristaux de L-lactate de magnésium obtenus sont **caractérisés par** une distribution de taille comprenant une taille médiane comprise entre environ 100 et environ 300 µm.

11. Procédé selon l'une quelconque des revendications 1 à 10, dans lequel la récupération des cristaux de L-lactate de magnésium est d'au moins 90 %.

12. Procédé selon l'une quelconque des revendications 1 à 11, dans lequel les cristaux de L-lactate de magnésium obtenus comprennent moins de 3 % de D-lactate de magnésium, de préférence moins de 2 % de D-lactate de magnésium, plus préférablement moins de 1,5 % de D-lactate de magnésium et idéalement moins de 1 % de D-lactate de magnésium.
